# EUROPÄISCHE PATENTSCHRIFT

(11) **EP 2 526 879 B1**
(45) Veröffentlichungstag und Bekanntmachung des Hinweises auf die Patenterteilung: **20.01.2016**
(21) Anmeldenummer: 12161729.4
(22) Anmeldetag: 28.03.2012
(51) Int. Cl.: A61B 17/16, A61B 17/88

(54) **Spannfutteranordnung einer medizinischen Handbohr- oder Handfräsmaschine**
Tensioning nut assembly of a medical hand-held drill or milling machine
Agencement de mandrin d'une machine de forage ou fraisage médicale

(30) Priorität: 24.05.2011 DE 102011050602
(43) Veröffentlichungstag der Anmeldung: 28.11.2012
(73) Patentinhaber: Aesculap AG, 78532 Tuttlingen (DE)
(72) Erfinder: Pfister, Ralph, 78647 Trossingen (DE); Mattes, Uwe, 78532 Tuttlingen (DE); König, Simone, 78048 VS-Villingen (DE)
(74) Vertreter: Winter, Brandl, Fürniss, Hübner, Röss, Kaiser, Polte - Partnerschaft mbB

(56) Entgegenhaltungen:
- DE-A1- 10 029 898
- DE-A1- 10 352 311
- DE-B4-102007 012 859
- DE-U1-202011 050 241

## Beschreibung

Die vorliegende Erfindung betrifft eine schlüssellose Spannfutteranordnung einer medizinischen Handbohr-/-fräsmaschine insbesondere gemäß dem Oberbegriff des Patentanspruchs 1.

Eine medizinische Handbohrmaschine ist ein elektrisches ggf. batteriegetriebenes, oder pneumatisches chirurgisches Instrument, welches beispielsweise zum Ausarbeiten von Montagelöchern in Knochenmaterial zur Fixierung von Implantaten Verwendung findet. In einer solchen Maschine werden spezielle Bohr- und/oder Fräswerkzeuge eingespannt, die auch während des Einsatzes vor Ort immer wieder gewechselt werden müssen. Dazu dienen manuell betätigbare Spann- oder Bohrfutter, deren Konstruktion ein festes Einspannen und Freigeben des Werkzeugs mit oder ohne Zuhilfenahme eines sogenannten Spannschlüssels gestatten. Ältere Maschinen verwenden sogenannte Zahnkranzbohrfutter, bei denen zum Spannen und Öffnen ein Spannschlüssel benötigt wird. Der Vorteil von Zahnkranzbohrfuttern besteht darin, dass Werkzeuge für beide Drehrichtungen (Rechts- und Linkslauf) sicher gespannt werden können. Andererseits sind Spannfutter mit Schlüssel heute aus ergonomischen und arbeitsökonomischen Gründen veraltet. Deshalb kommen heute insbesondere auch schlüssellose Bohr-/Spannfutter zum Einsatz.

Das Dokument DE10352311 A1 offenbart eine schlüssellose Spannfutteranordnung gemäß dem Oberbegriff des Patentanspruchs 1.

Aus dem Stand der Technik beispielsweise gemäß der DE 10 2007 012 859 B4 ist eine schlüssellose Spannfutteranordnung dieser Gattung bekannt. Diese betrifft eine Spannfuttereinheit bestehend aus einem mehrteiligen Gehäuse, in dem eine Trägerhülse (Antriebswelle) drehbar, jedoch axial fest gelagert ist. An dieser Trägerhülse ist an einem aus dem Gehäuse axial herausstehenden (proximalen) Hülsenendabschnitt ein Halte-/Griffring fest fixiert. In dem Gehäuse ist ferner ein Spannbackenträger mit konisch zulaufendem vorderem Endbereich drehfest montiert, in dem Gleitführungen für eine Anzahl von Spannbacken ausgeformt sind. D.h., der Spannbackenträger und das Gehäuse bestehend aus einer Mantelhülse und einer Verschlusshülse sind drehfest miteinander verbunden. Axial zwischen dem Spannbackenträger und der Trägerhülse ist eine Druckspindel angeordnet. Die Druckspindel hat an ihrem Außenumfang ein Spindelgewinde und ist hiermit in die Trägerhülse eingeschraubt. An der dem Spindelgewinde abgewandten Stirnseite besitzt die Druckspindel Nuten für die Aufnahme der Spannbacken.

Um die Spannfuttereinheit zu öffnen, wird der Griffring mit der einen Hand und das Gehäuse (auch Verschlusshülse genannt) vorzugsweise im Bereich der Spannbacken mit der anderen Hand festgehalten und jeweils entgegengesetzt zueinander (d.h. das Gehäuse gegen den Uhrzeigersinn und der Griffring im Uhrzeigersinn) gedreht. Dadurch wandert die Druckspindel entsprechend der Ganghöhe des Spindelgewindes in die Trägerhülse hinein und zieht dabei die Spannbacken in die gleiche Richtung zurück. Aufgrund der Konizität des vorderen Gehäuseabschnitts und der Führung des Backenträgers bewegen sich die Spannbacken gleichzeitig radial auseinander. Auf diese Weise erweitert sich der von den Spannbacken definierte Aufnahmedurchmesser. Soll die Spannfuttereinheit geschlossen werden, muss lediglich das Gehäuse unter Festhalten des Griffrings nunmehr in Uhrzeigerrichtung gedreht werden, wodurch sich die Druckspindel aus der Trägerhülse heraus bewegt und die Spannbacken vorwärts schiebt. Dabei verengt sich der Aufnahmedurchmesser wieder, wodurch das zuvor eingesteckte Werkzeug eingespannt wird.

Das Spindelgewinde ist so konstruiert, dass es selbsthemmend wirkt. Zudem ist die Gewinderichtung so gewählt, dass im normalen Gebrauch der Maschine, vorliegend eine Rotation im Uhrzeigersinn (Rechtslauf), eine zusätzliche Einspannkraft (-moment) auf die Trägerhülse aufgebracht wird, die ein weiteres Festspannen des Werkzeugs in der Spannfuttereinheit bewirkt. In anderen Worten ausgedrückt, sind schlüssellose Spannfutteranordnungen, welche auf dem vorstehend genannten Druckspindel-Prinzip beruhen, bauartbedingt im Wesentlichen nur für eine Drehrichtung (in der Regel Rechtslauf-Betrieb) geeignet. Ist die schlüssellose Spannfutteranordnung zweihülsig, d.h. bestehend aus Gehäuse (oder Verschlusshülse) und Griffring ausgeführt, ist das Handling beim Spannen und Öffnen zudem immer noch sehr umständlich. Der Benutzer benötigt nämlich in diesem Fall beide Hände an der Spannfutteranordnung für das gegeneinander Drehen von Gehäuse und Griffring, wobei er gleichzeitig aber auch noch die Maschine (einschließlich Motor) irgendwie halten muss.

Trotz dieser Schwierigkeiten hat die schlüssellose Spannfutteranordnung gegenüber einem altbekannten Zahnkranzbohrfutter den großen Vorteil, dass zum Spannen und Lösen kein externes Bedienerwerkzeug erforderlich ist, welches für einen Patienten sogar eine Gefahr darstellen kann, wie dies nachstehend kurz erläutert wird.

Bei chirurgischen Eingriffen ist die Spannfuttereinheit an die Bohr- und Fräsmaschine angekuppelt. Während einer Operation ist es aber häufig notwendig, dass die Werkzeuge mehrmals gewechselt werden müssen. Es kann auch vorkommen, dass ein Werkzeug im OP-Situs verbleibt und später wieder aufgenommen wird. Dies erfordert ein wiederholtes Spannen und Lösen der Spannfuttereinheit. Bei diesem Vorgang wird die nachteilige Handhabung eines Zahnkranzbohrfutters erkennbar. Ist nämlich ein Spannschlüssel erforderlich, muss zum Einen die Bohr- und Fräsmaschine und zum Anderen der Spannschlüssel gehalten und gedreht werden. Während des Hantierens mit dem Spannschlüssel werden Maschine und Werkzeug unvermeidbar geschwenkt und gekippt, sodass das ausgearbeitete Montageloch in dem das Werkzeug ggf. noch steckt, ausbrechen kann.

Im Fall einer schlüssellosen Spannfutteranordnung verbessert sich zwar diese Situation, aber auch hier müssen zumindest Gehäuse und Haltering zumindest nach derzeitigem Stand der Technik gegeneinander verdreht werden, was mit zwei Händen ebenfalls schwierig ist. Insofern ist auch das Handling der bekannten schlüssellosen Spannfutteranordnung unbefriedigend.

Ein anderes Problem besteht darin, dass beispielsweise zum Linksgewinde schneiden, Herausdrehen von Schrauben, etc. ein Linkslauf erforderlich ist. Eine schlüssellose Spannfutteranordnung (-einheit) beispielsweise der Drei-Backen-Bauart mit Druckspindel kann sich aber im Linkslauf öffnen. Geschieht dies während einer Operation, kann das Werkzeug im Knochen stecken bleiben. Um dieses dann wieder zu bergen, werden meistens zwei Personen benötigt. Die eine Person muss die Maschine genau über dem stecken gebliebenen Werkzeug ausrichten und die andere Person muss das Werkzeug erneut einspannen. Dies kostet Zeit und erhöht das Operationsrisiko für den Patienten.

Angesichts dieser komplexen Problematik des Stands der Technik ist es die Aufgabe der vorliegenden Erfindung, eine schlüssellose Spannfutteranordnung (-einheit) einer Schraub-, Bohr- und/oder Fräsmaschine vorzugsweise der Handmaschinenbauart bereit zu stellen, die eine höhere Funktionalität aufweist. Ein Ziel ist es, die gattungsgemäße Maschine sowohl rechts- als auch linksläufig sicher einsetzen zu können. Ein weiteres Ziel ist es, den Spannvorgang zu vereinfachen und so einen Werkzeugwechsel auch von einer Person problemlos durchführbar zu machen.

Die vorstehend genannte Aufgabe wird durch eine schlüssellose Spannfutteranordnung (-einheit) vorzugsweise mit drei Spannbacken und Druckspindelprinzip mit den Merkmalen des Anspruchs 1 gelöst. Vorteilhafte Ausgestaltungen der Erfindung sind Gegenstand der Unteransprüche.

Der Grundgedanke der Erfindung besteht demzufolge darin, durch Anordnung einer bei einer Drehrichtung entgegen der vorbestimmten Hauptdrehrichtung wirkenden sowie vorzugsweise manuell oder automatisch einrückenden Bremse (oder Sperrkupplung) ein Verdrehen des Druckspindelantriebs bezüglich des Spannfuttergehäuses (bzw. der Verschlusshülse) für ein unbeabsichtigtes Öffnen der Spannfutteranordnung zu blockieren. Ein anderer oder zusätzlicher Grundgedanke der Erfindung besteht in der Vorschaltung eines Über- oder Untersetzungsgetriebes (Vorgelege) mit integrierter Wellenarretierung, wodurch die Anordnung eines Griff-/Halterings auf der Antriebswelle überflüssig wird. In diesem Fall kann nämlich ein Gegendrehmoment zum manuell an die Verschlusshülse angelegten Öffnungsmoment über das Gehäuse der Bohr- und/oder Fräsmaschine aufgebracht werden, sodass ein Umgreifen vom Maschinengriff auf den (nicht mehr vorhandenen) Haltering entfällt.

Ein konkreter Aspekt der Erfindung sieht demzufolge die Bereitstellung einer (vorzugsweise) schlüssellosen Spannfutteranordnung einer medizinischen Bohr- und/oder Fräsmaschine vorzugsweise in der Form einer Spannfuttereinheit vor, mit einem Spannfuttergehäuse, in dem (weiter vorzugsweise drei) Spannbacken mittels einer Druckspindel axialverschiebbar sind, die mit einer im Spannfuttergehäuse drehbar gelagerten Antriebswelle der Spannfuttereinheit in Gewinde- bzw. Spindeleingriff steht. Erfindungsgemäß ist ein wahlweise (vorzugsweise manuell oder automatisch) aktivier- und/oder deaktivierbarer Freilauf (als die Bremse oder Sperrkupplung) zwischen der Antriebswelle und dem Spannfuttergehäuse eingebaut. Der Freilauf wird (manuell oder automatisch) aktiviert, wenn ein Bohr- und/oder Fräsbetrieb aufgenommen wird. In diesem Fall erlaubt der Freilauf bei einem Betrieb in Hauptdrehrichtung, z.B. Rechtslauf, ein weiteres Festziehen der Spannfuttereinheit in Übereinstimmung mit dem Stand der Technik. Im Unterschied hierzu blockiert jedoch der Freilauf eine Relativbewegung zwischen Spannfuttergehäuse und Antriebswelle für ein ungewolltes Öffnen/Lockern der Spannfuttereinheit im Fall eines gegenläufigen Betriebs in Nebendrehrichtung, z.B. Linkslauf. Soll die Spannfuttereinheit geöffnet werden, wird hierfür der Freilauf (manuell oder automatisch) deaktiviert, sodass eine Relativdrehbewegung der Antriebswelle bezüglich des Gehäuses entgegen der Hauptdrehrichtung ermöglicht wird.

Vorteilhaft ist es hierbei, wenn zwischen dem Freilauf und dem Spannfuttergehäuse oder zwischen dem Freilauf und der Antriebwelle eine vorzugsweise manuell (oder automatisch) betätigbare Kupplung angeordnet ist. Über diese kann der Freilauf dem (über die Spannbacken geführten) Drehmomentfluss optional für dessen Aktivierung parallel geschaltet werden. Alternativ ist es aber auch möglich, eine derartige Kupplung beispielsweise mit dem Wahlschalter für Rechts- /Linkslauf zu koppeln und den Freilauf quasi automatisch erst dann zu aktivieren, wenn der Wahlschalter aus der Hautdrehrichtungsposition (z.B. Rechtslauf) in die Nebendrehrichtungsposition (z.B. Linkslauf) umgeschaltet wird. Schließlich besteht die Möglichkeit einer vollautomatischen Kupplung, welche bei (motorisiertem) Antreiben der Spannfuttereinheit (beispielsweise mit Betätigen des Motor-Startschalters) den Freilauf aktiviert.

Gemäß einem anderen oder zusätzlichen Aspekt der Erfindung ist der Freilauf ein auf der Antriebswelle oder im Spannfuttergehäuse reibschlüssig fixierbarer Hülsenfreilauf und die Kupplung ein zwischen den Hülsenfreilauf und das Spannfuttergehäuse oder zwischen den Hülsenfreilauf und die Antriebswelle axial einschiebbares Drehmoment-Übertragungsbauteil, über das die reibschlüssige Fixierung des Hülsenfreilaufs aktiviert werden kann. Vorteilhaft ist es hierbei, wenn das Drehmoment-Übertragungsbauteil ein Formschlusselement, beispielsweise ein (Nut- und) Federelement oder eine den Hülsenfreilauf umgreifende sowie diesen fest aufnehmende sowie mit der Antriebswelle oder dem Spannfuttergehäuse formschlüssig gekoppelte Schiebehülse ist, wobei das Formschlusselement weiter vorzugsweise in Axialrichtung der Antriebswelle zwischen einer Eingriffs- und Freigabeposition des Hülsenfreilaufs hin- und herbewegbar ist.

Vorteilhaft ist es hierbei, wenn der Hülsenfreilauf zusammen (quasi einstückig) mit dem Drehmoment-Übertragungsbauteil verschoben wird, um so in die bzw. aus der vorstehend genannten Reibschlussposition zu kommen, während der Formschluss des Drehmoment-Übertragungsbauteils permanent beibehalten bleibt. Ein solcher rein mechanischer Aufbau ist einfach in seiner Funktion und damit vergleichsweise robust und wenig störanfällig. Auch ist seine Handhabung unkompliziert und übersichtlich, sodass das Risiko einer Fehlbedienung während der Operation gering ist. Auch wird das Aktivieren und Deaktivieren des Freilaufs (infolge der Reibschlussverbindung) unabhängig von der Drehposition der Antriebswelle relativ zum Spannfuttergehäuse, sodass ein Verklemmen der Kupplung bei deren Betätigung ausgeschlossen ist.

Weiter vorteilhaft ist es, wenn die Schiebehülse einen Rastvorsprung oder Rastrücksprung hat, der mit einem entsprechenden Gegenstück (bestehend aus zwei axial beabstandeten Rücksprüngen oder Vorsprüngen) auf Seiten der Antriebswelle oder des Spannfuttergehäuses zusammenwirkt, um dadurch zwei Rastpositionen festzulegen, von denen die eine Rastposition eine Reibschlussstellung für ein Aktivieren des Freilaufs (Eingriffsposition) und die andere Rastposition eine Gleit- bzw. Durchrutschstellung für ein Deaktivieren des Freilaufs (Freigabeposition) definiert. Durch diese Rastung wird dem Bediener das korrekte Einnehmen der gewählten Stellung (haptisch und ggf. akustisch) signalisiert, wodurch die Bedienbarkeit verbessert wird.

Eine Variante sieht hierbei vor, die Schiebehülse manuell zu betätigen. Es ist aber auch möglich, die Schiebehülse in einer spiralförmigen Kulisse an der Umfangsseite der Antriebswelle zu führen, sodass eine Drehung der Antriebswelle zwangsläufig in einer Axialverschiebung der Schiebehülse resultiert. Unabhängig davon kann die Schiebehülse aber auch elektrisch/elektromagnetisch betätigt werden.

Ein anderer oder zusätzlicher Aspekt der Erfindung sieht die Anordnung einer Getriebeeinrichtung mit integrierter (automatisch wirkender) Wellenarretierung vor, die als externe (separate) Einheit der vorstehend beschriebenen Spannfuttereinheit vorgeschaltet bzw. vorschaltbar ist, d.h. die zwischen eine Abtriebswelle der Bohr- und/oder Fräsmaschine und die Antriebswelle im Spannfuttergehäuse wahlweise einsetzbar oder eingesetzt ist. Die Getriebeeinrichtung bzw. -einheit übernimmt somit zwei Funktionen, nämlich das Über-/Untersetzen der von der Bohr- und/oder Fräsmaschine erzeugten Drehzahl in eine werkzeugspezifische Drehzahl und das Festhalten der Antriebswelle im Spannfuttergehäuse während des manuellen Spannens oder Lösens der Spannfuttereinheit als Ersatz für einen (dann) nicht mehr notwendigen Haltering, wie er aus dem Stand der Technik bekannt ist.

Eine vorteilhafte Ausgestaltung der Erfindung sieht hierfür vor, dass die Getriebeeinrichtung bzw. -einheit eine in einem Getriebegehäuse gelagerte Eingangswelle hat, die über einen Zahnrad-Getriebezug ein erstes Klauenkupplungsstück (Mitnahmeelement) antreibt, das mit einem zweiten Klauenkupplungsstück (Abtriebselement) in Kupplungseingriff steht, welches auf einer im Getriebegehäuse gelagerten Ausgangswelle der Getriebeeinrichtung drehfest sitzt oder einstückig mit dieser ausgebildet ist. Weiter vorteilhaft ist es, wenn das zweite Klauenkupplungsstück in Umfangsabschnitten zwischen seinen axial vorragenden Klauen (weitgehend plane) Umfangsflächen (Klemmflächen) ausbildet, die zwischen sich und einer Innenwand des Getriebegehäuses jeweils einen im Querschnitt vorzugsweise kreissegmentförmigen Aufnahmeraum definieren, in den jeweils ein Wälzkörper eingesetzt ist, der sich bei einer aktiven (vorzugsweise manuellen) Verdrehung des zweiten Klauenkupplungsstücks relativ zum Getriebegehäuse zwischen der jeweiligen planen Umfangsfläche und der Innenwand verspannt. D.h. die Klauenkupplung hat oder ist gleichzeitig die Wellenarretierung, die dann, wenn das zweite Klauenkupplungsstück aktiv, d. h. bei einem manuellen Verdrehen des Spannfuttergehäuses beispielsweise für ein Lösen (aber auch Verspannen) der Spannfuttereinheit gedreht wird, genau diese Drehung unmittelbar auf das Gehäuse der Getriebeeinheit überträgt, das wiederum mit dem Gehäuse der Bohr- und/oder Fräsmaschine fest verbunden ist. Mit Festhalten des Maschinengehäuses an einem dafür vorgesehenen Maschinengriff, wird somit gleichzeitig auch die Klauenkupplung und damit die Antriebswelle der Spannfuttereinheit festgehalten.

Eine weitere oder andere vorteilhafte Weiterbildung sieht vor, dass das erste Klauenkupplungsstück axial vorragende Klauen hat, die so dimensioniert sind, dass sie bei einer aktiven Verdrehung des ersten Klauenkupplungsstücks relativ zum Getriebegehäuse (d.h. bei regulärem, motorisiertem Betreiben der Schraub-, Bohr- und/oder Fräsmaschine) für ein Antreiben des zweiten Klauenkupplungsstücks die Wälzkörper mitnehmen, um deren Verspannen zwischen der jeweiligen planen Umfangsfläche (oder Klemmfläche) und der Innenwand des Getriebegehäuses zu vermeiden. In diesem Fall ist die Wellenarretierung inaktiv und eine leichtgängige Drehung der Klauenkupplung möglich.

Ein anderer oder zusätzlicher Aspekt der Erfindung sieht vor, dass der Zahnrad-Getriebezug ein Planetengetriebe, vorzugsweise der Doppelplanetengetriebebauart (zweistufiges Planetengetriebe) ist mit einem (einzigen) Hohlrad (-element), das büchsenförmig in das Getriebegehäuse drehfest eingesetzt ist und gleichzeitig die (Gehäuse-) Innenwand für die Wälzkörper bildet. Dies hat den Vorteil, dass die Klemmkraft ausgehend von den Wälzkörpern in das Hohlradmaterial eingeleitet wird, das aus einem speziellen Lager-Material und/oder gehärtet ist und damit diesen (linienförmig) von den Wälzkörpern aufgebrachten Klemmkräften standhalten kann. Da das Hohlrad über eine große Fläche mit dem eigentlichen Getriebegehäuse Kontakt hat, können so die punktuellen Klemmkräfte gut in das Getriebegehäuse flächig verteilt werden. Dieses wird daher weniger belastet und kann demzufolge aus einem leichteren/weniger belastbaren Material hergestellt sein.

Abschließend sei darauf hingewiesen, dass die Merkmale gemäß der vorstehend genannten Aspekte der Erfindung individuell die gestellte Aufgabe lösen bzw. einen Beitrag zur Lösung der Aufgabe leisten und daher separat oder in Kombination miteinander beanspruchbar sind.

Die Erfindung wird nachstehend anhand eines bevorzugten Ausführungsbeispiels unter Bezugnahe auf die begleitenden Figuren näher beschrieben.
Fig. 1 zeigt den Längsschnitt einer Spannfutteranordnung und insbesondere einer separaten (individuellen) Spannfuttereinheit der Spannfutteranordnung gemäß dem bevorzugten Ausführungsbeispiel der Erfindung,
Fig. 2 zeigt den Längsschnitt einer separaten (individuellen) Getriebeeinheit mit integrierter Wellenarretierung der Spannfutteranordnung gemäß dem bevorzugten Ausführungsbeispiel der Erfindung,
Fig. 3 zeigt eine Explosionsdarstellung der Wellenarretierung gemäß Fig. 2 und
Fig. 4 zeigt einen Querschnitt der Wellenarretierung gemäß der Fig. 2 und 3 in gelöster (inaktiver) Stellung (rechte Abbildung) und in arretierter/verspannter (aktiver) Stellung (linke Abbildung).

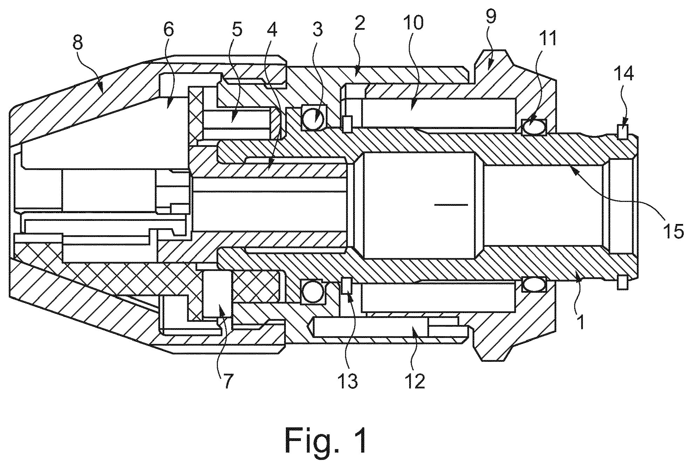

In der Fig.1 ist die Konstruktion einer Spannfuttereinheit der erfindungsgemäßen Spannfutteranordnung als ein bevorzugtes Ausführungsbeispiel im Längsschnitt dargestellt. Die Spannfuttereinheit ist demzufolge nach dem Druckspindelprinzip aufgebaut und hat insbesondere im vorliegenden Fall eine vorzugsweise hohl-/teilhohlzylindrische Antriebswelle 1, die in einem Spannfuttergehäuse, insbesondere einer Mantelhülse 2 als Teil des Spannfuttergehäuses über Lager 3 (in Fig. 1 ist lediglich ein Kugellager 3 schematisch dargestellt) drehbar, jedoch axial fest gehalten ist. An einem proximalen, einer nicht weiter dargestellten Bohr-/Fräsmaschine zugewandten Endabschnitt ragt die Antriebswelle 1 aus der Mantelhülse 2 heraus.

In einem distalen, der Schraub-/Bohr-/Fräsmaschine abgewandten, jedoch noch innerhalb der Mantelhülse 2 liegenden Endabschnitt hat die Antriebswelle 1 ein inneres Spindelgewinde, in das eine Druckspindel 4 axialseitig eingedreht ist. Die Druckspindel 4 ist an ihrem dem Spindelgewinde abgewandten Ende in einem Backenträger 5 axialverschiebbar, der in die Mantelhülse 2 an deren distaler Stirnseite eingesetzt und mittels einer Anzahl von radialen Bolzen/Stiften 7 darin fixiert ist.

Der Backenträger 5 ist an seinem distalen Endabschnitt konisch geformt und weist axial sich erstreckende Führungsnuten und/oder Schienen auf (nicht im Einzelnen gezeigt), an denen platten/plättchenartige Spannbacken 6 (vorzugsweise drei Spannbacken 6) axialverschiebbar gehalten sind. Die der Druckspindel 4 jeweils zugewandten (schmalen) Stirnkanten der Spannbacken 6 stützen sich axial auf der Stirnseite der Druckspindel 4 ab, die hierfür nicht weiter dargestellte Radialnuten aufweist, in denen die Spannbacken 6 radial gleitfähig gehalten sind. Dabei bildet der Backenträger 5 an seinem proximalen Axialabschnitt einen radial umlaufenden äußeren Vorsprung (Ringvorsprung) aus, der zum Einen als Axialflansch gegenüber der Mantelhülse 2 und zum Anderen als axialer Endanschlag zum Begrenzung der Axialbewegung der Spannbacken 6 für ein Öffnen der Spannfuttereinheit dient.

Der am distalen Endbereich konusförmig sich verjüngende Backenträger 5 ist ferner in diesem Axialabschnitt von einer Verschlusshülse 8 umgeben, die fest mit der Mantelhülse 2 verschraubt oder anders fest verbunden ist und somit zusammen mit der Mantelhülse 2 das Spannfuttergehäuse der Spannfuttereinheit bildet.

Am proximalen Endabschnitt des Spannfuttergehäuses ist eine wahlweise ein-/ausrückbare Kupplungseinrichtung vorzugsweise in Form eines Freilaufs angeordnet.

Diese besteht aus einem schaltbaren Drehmoment-Übertragungsbauteil vorliegend eine Schiebehülse 9, die axialverschieblich auf der Antriebswelle 1 und/oder in der Mantelhülse 2 gehalten ist, derart, dass sie eine vorbestimmte, durch Rast- oder Anschlagspositionen festgelegte Axialstrecke in die und aus der Mantelhülse 2 verschiebbar ist.

Zwischen der Schiebehülse 9 und der Antriebswelle 1 ist ein Hülsenfreilauf 10 eingesetzt. Der Hülsenfreilauf 10 ist hierfür außenumfangsseitig in die Schiebehülse 9 reib- und/oder formschlüssig fest eingepresst oder eingesetzt, um sich gemeinsam (einstückig) mit dieser axial verschieben zu können. Die radiale Innenseite des Freilaufs 10 ist axial gleitend auf der Mantelfläche der Antriebswelle 1 geführt.

Je nach Axialposition der Schiebehülse 9 geht der Freilauf 10 eine maßgeblich in Umfangsrichtung wirkende Reibschlussverbindung mit der Antriebswelle 1 ein. Dies kann z.B. dadurch bewirkt werden, indem die Antriebswelle 1 außenumfangsseitig im Bereich des axialen Schiebewegs der Schiebehülse 9 konisch sich aufweitend ausgebildet ist, sodass der Freilauf 10 schiebewegabhängig auf der Antriebswelle 1 reibschlüssig aufsitzt bzw. sich von dieser löst. Die Schiebehülse 9 hat an ihrem Außenumfang eine oder mehrere Axialnuten oder eine Nut- und Federanordnung, um mit der radialen Innenseite der Mantelhülse 2 einen in Umfangsrichtung wirkenden, jedoch eine Axialverschiebung erlaubenden Formschluss einzugehen.

Alternativ zu dieser in Fig. 1 dargestellten Konstruktion kann der Hülsenfreilauf 10 an seinem Innenumfang mit einer oder mehreren Axialnut(en) oder einer Nut- und Federanordnung ausgebildet sein und die Antriebswelle 1 mit einem oder mehreren äußeren Axialstegen (Nut und Federn) entsprechend versehen sein, um mit dem Hülsenfreilauf 10 einen drehfesten Formschluss einzugehen. Auch eine umgekehrte Anordnung der Nuten und Stege (Federn) ist natürlich denkbar. Die Schiebehülse 9 geht in diesem alternativen (nicht dargestellten) Beispiel mit der Mantelhülse 2 ebenfalls einen (permanenten) Formschluss ein, wofür sowohl die Schiebehülse 9 an ihrem äußeren Umfang als auch die Mantelhülse 2 an ihrem inneren Umfang jeweils mit zumindest einer Längsnut versehen sind, in die der Federstift 12 eingesetzt ist. Alternativ hierzu ist aber auch ein Vielnutprofil oder dergleichen Formschlussverbindung denkbar. Die innere Umfangsfläche der Schiebehülse 9 stellt hingegen in Abhängigkeit der Axialstellung wahlweise eine Reibschlussverbindung mit dem Hülsenfreilauf 10 her, wofür die Schiebehülse 9 und/oder der Hülsenfreilauf 10 mit einem Reibbelag versehen sein kann.

Entscheidend bei der Freilaufkonstruktion ist, dass der Hülsenfreilauf 10 zumindest mit der Antriebswelle 1 oder der Mantelhülse 2 einen Axialpositionsabhängigen Reibschluss eingeht und vorzugsweise mit dem jeweils anderen Bauteil, nämlich der Mantelhülse 2 oder der Antriebswelle 1 einen permanenten Formschluss in Umfangsrichtung hat. Dadurch wird das Aktivieren und Deaktivieren des Freilaufs unabhängig von der Relativ-Drehposition der Antriebswelle 1 zur Mantelhülse 2.

Wie in der Fig. 1 ferner dargestellt ist, hat die Schiebehülse 9 zudem an ihrem proximalen Endabschnitt einen den Hülsenfreilauf 10 stirnseitig umgreifenden Flansch, der gleitend an der äußeren Umfangsfläche der Antriebswelle 1 abschließt und einen Federring 11 trägt, der bei Axialverschieben der Schiebehülse 9 längs der Antriebswelle 1 elastisch abgleitet. Die Antriebswelle 1 ist zudem mit zwei axial beabstandeten Vorsprüngen/Rücksprüngen ausgebildet (in der Fig. 1 sind zwei axial beabstandete Rücksprünge in Form von Welleneindrehungen an der Antriebswelle 1 gezeigt), in welche der Federring 11 bei Übergleiten einschnappt und somit jeweils eine Schiebeendposition definiert. Um ein Abziehen der Schiebehülse 9 zu vermeiden ist schließlich ein Sicherungsring 14 auf der Antriebswelle 1 aufgesetzt, der einen Endanschlag für die Schiebehülse 9 bildet.

Die eine Endposition, definiert durch eine Welleneindrehung, bestimmt somit die maximal aus der Mantelhülse 2 ausgefahrene Position der Schiebehülse 9, in der kein Reibschluss möglich ist und der Freilauf somit deaktiviert wird, wohingegen die andere Endposition, definiert durch die andere Welleneindrehung, die maximal in die Mantelhülse 2 eingefahrene Position der Schiebehülse 9 bestimmt, in der ein maximaler Reibschluss erreicht wird und der Freilauf folglich aktiviert ist.

Abschließend sei noch darauf verwiesen, dass die Antriebswelle 1 an ihrem proximalen, aus der Mantelhülse 2 herausragenden Endabschnitt mit einem Innengewinde 15 versehen ist, in das eine weitere Antriebswelle (in der Fig. 1 nicht weiter gezeigt) von Seiten eines Antriebsmotors eingeschraubt werden kann. Alternativ hierzu ist aber auch eine Steckverbindung oder eine andere Wellenkupplung bekannter Bauart verwendbar.

Die Funktionsweise der Spannfuttereinheit als ein (individueller) Bestandteil der erfindungsgemäßen Spannfutteranordnung soll nachfolgend erläutert werden.

Für das Einsetzen eines Werkzeugs, z.B. ein Bohrer oder Fräser, muss zunächst die von den Spannbacken 6 bestimmte "Schlüsselweite" vergrößert werden. Zu diesem Zweck wird zunächst die Schiebehülse 9 aus der Mantelhülse 2 axial herausgezogen und somit der Reibschluss zwischen dem Freilauf 10 und der Antriebswelle 1 unterbrochen. In dieser Position ist der Freilauf demnach inaktiv geschaltet, sodass die Antriebswelle 1 gegenüber der Mantelhülse 2 auch in Sperrrichtung des Freilaufs 10 gedreht werden kann. Nun wird die Antriebswelle 1 festgehalten (entweder manuell über den Haltering oder automatisch über eine integrierte Wellenarretierung) und das Spannfuttergehäuse in die Richtung gedreht, die ein Einschrauben der Druckspindel 4 in die Antriebswelle 1 bewirkt. Dabei werden die Spannbacken 6 kontinuierlich längs des Backenträgers 5 zurückgezogen und bewegen sich dabei gleichzeitig infolge der Konizität des Backenträgers 5 und der Verschlusshülse 8 radial auseinander.

Soll ein eingesetztes Werkzeug zwischen den Spannbacken 6 eingespannt werden, muss bei festgehaltener Antriebswelle 1 das Spannfuttergehäuse (an der Verschlusshülse 8) in jene Richtung gedreht werden, die ein Ausdrehen der Druckspindel 4 aus der Antriebswelle 1 bewirkt, wodurch die Spannbacken 6 kontinuierlich unter Einengung der "Schlüsselweite" längs des Backenträgers verschoben werden, solange, bis eine ausreichende radiale Spannkraft auf das Werkzeug aufgebracht ist. Schließlich wird die Schiebehülse 9 in die Mantelhülse 2 axial eingeschoben und dabei zur Aktivierung des Freilaufs 10 ein Reibschluss zwischen dem Hülsenfreilauf 10 und der Antriebswelle 1 hergestellt.

Im Betrieb bei einer Betätigung in Hauptdrehrichtung (z.B. Rechtslauf) bringt die Antriebswelle 1 über die Druckspindel 4 und die Spannbacken 6 ein Drehmoment auf das Werkzeug auf, das in eine Richtung wirkt, in der die Druckspindel 4 aus der Antriebswelle 1 herausgedreht wird. Da der Freilauf 10 in diese Richtung ein Verdrehen zwischen Antriebswelle 1 und Spannfuttergehäuse erlaubt, bewirkt dies ein weiteres Festziehen der Spannbacken 6. Bei einer Betätigung in Nebendrehrichtung (z.B. Linkslauf) würde dies hingegen bedeuten, dass die Antriebswelle 1 über die Druckspindel 4 und die Spannbacken 6 ein Drehmoment auf das Werkzeug aufbringt, das in eine Richtung wirkt, in der die Druckspindel 4 in die Antriebswelle 1 hineingedreht wird. Da der Freilauf 10 in diese Richtung jedoch ein Verdrehen zwischen Antriebswelle 1 und Spannfuttergehäuse sperrt, wird der Drehmomentfluss in diesem Fall parallel zu der Druckspindel 4 von der Antriebswelle 1 über den Freilauf 10 und das Spannfuttergehäuse in den Backenträger 5 und von dort in die Spannbacken 6 geführt. Dies bewirkt zwar kein weiteres Festziehen der Spannbacken 6, aber auch kein Lösen der Spannbacken 6. Damit wird die schlüssellose Spannfuttereinheit gemäß dem bevorzugten Ausführungsbeispiel der Erfindung für den Betrieb in Nebendrehrichtung gemäß vorstehender Definition tauglich.

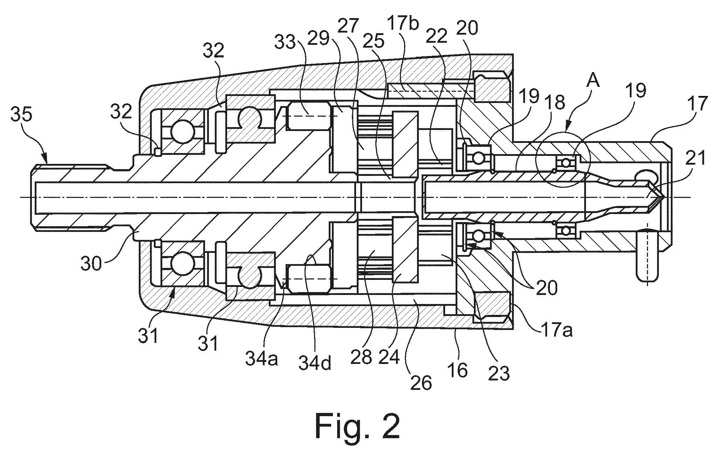

In den Fig. 2 bis 4 ist eine als separate (individuelle) Einheit zusammengefasste Getriebeeinrichtung gemäß dem bevorzugten Ausführungsbeispiel der Erfindung dargestellt.

Insbesondere gemäß der Fig. 2 hat diese Getriebeeinheit ein mehrteiliges Getriebegehäuse bestehend aus einer im Wesentlichen hülsenförmigen Kartusche 16, in die stirnseitig ein Montageflansch 17 dichtend (durch eine Dichtung 17a) sowie mittels einer Nut- und Federverbindung 17b drehfest eingesetzt (verschraubt) ist, der wiederum mit dem Gehäuse der nicht weiter dargestellten Bohr-/Fräsmaschine in Steck- und/oder Rasteingriff bringbar ist.

Innerhalb des Montageflansches 17 ist eine Eingangswelle 18 über Kugellager 19 drehbar gelagert, die mittels Wellenringe 20 axial gesichert sind. Die Eingangswelle 18 hat an ihrem proximalen Ende eine Wellenkupplung 21, die mit der Abtriebswelle der Maschine in Dreheingriff bringbar ist. An dem distalen Ende der Eingangswelle 18 ist ein Zahnrad 22 fixiert oder ausgebildet, welches das Eingangselement eines Zahnradgetriebes, vorliegend z.B. ein Doppelplanetengetriebe (zweistufiges Planetengetriebe) darstellt.

Im Konkreten ist an der Eingangswelle 18 ein erstes Sonnenrad 22 drehfest fixiert, das mit ersten Planetenrädern 23 kämmt, die an einem ersten drehbaren Planetenradträger 24 mit daran fixierter Zwischenwelle 25 gelagert sind und an einem drehfesten Hohlrad 26 abwälzen. Das Hohlrad 26 ist hierbei als eine Hülse vorzugsweise aus speziellem Lagermaterial und/oder gehärtet ausgeformt, die drehfest über eine Nut- und Federverbindung in die Kartusche 16 eingesetzt und durch den Flansch 17 axial gesichert ist. Auf der Zwischenwelle 25 sitzt ferner drehfest ein zweites Sonnenrad 27, das mit zweiten Planetenrädern 28 in Kämmeingriff ist und die ebenfalls am gleichen Hohlrad 26 abwälzen. Die zweiten Planetenräder 28 sind an einem zweiten Planetenradträger 29 gelagert, der relativ (gleitend) verdrehbar auf einer Ausgangswelle 30 der Getriebeeinheit sitzt. An dieser Stelle sei noch darauf hingewiesen, dass der erste Planetenradträger 24 mit daran angeschlossener Zwischenwelle 25 durch den Kämmeingriff zwischen den Planetenrädern 23 und 28, den Sonnenrädern 22 und 27 sowie dem Hohlrad 26 drehbar gehalten wird. Es besteht aber theoretisch auch die Möglichkeit, dass die Zwischenwelle 25 als Hohlwelle ausgeführt und auf einem axialen Endabschnitt der Ausgangswelle 30 relativ drehbar gelagert ist.

Die Ausgangswelle 30 ist wiederum über Kugellager 31 in der Kartusche 16 drehbar abgestützt sowie mittels Wellenringe 32 axial fixiert.

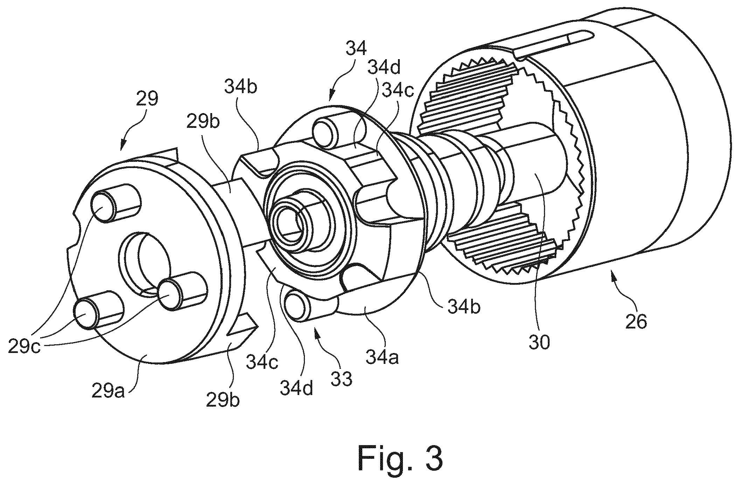

Der in Fig. 3 perspektivisch gezeigte zweite Planetenradträger 29 bildet ein Mitnahmeelement des zweistufigen Getriebes in der Form eines Klauenkupplungsstücks, insbesondere bestehend aus einer Kreisscheibe oder -platte 29a, an der auf einer Scheibenseite eine Anzahl (vorliegend vier) Klauen 29b axial sich ersteckend angeordnet sind, wohingegen auf der anderen Scheibenseite sich Lagerzapfen 29c für die zweiten Planetenräder 28 befinden.

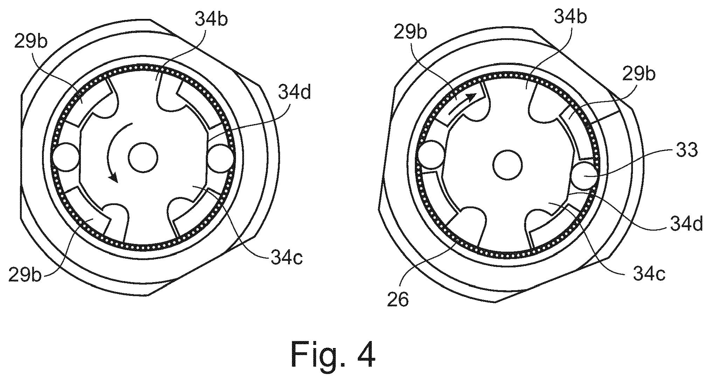

Auf der Ausgangswelle 30 der Getriebeeinheit sitzt drehfest (oder ist einstückig daran ausgeformt) ein Abtriebselement in der Form eines weiteren Klauenkupplungsstücks 34 bestehend aus einer Kreisscheibe oder -platte 34a, an der sich axial vorragende Klauen 34b ausformen (vorliegend zwei Klauen), die in einem Winkelabstand zueinander (vorliegend 180°) auf einer Umlaufbahn angeordnet sind. In den Winkelabständen bildet das Klauenkupplungsstück (bzw. Abtriebselement) 34 gemäß der Fig. 3 (vorliegend zwei) radial sich erstreckende Sockel 34c aus, die an ihren Umfangsseiten radial nach außen weisende, nahezu plane Flächenbereiche oder Klemmflächen 34d ausformen. In eingebautem Zustand dieses Klauenkupplungsstücks (bzw. Abtriebselements) 34 gemäß der Fig. 4 begrenzt jede der planen Flächenbereiche 34d zusammen mit der das Hohlrad ausbildenden Hülse 26 einen kreissegmentförmigen Hohlraum, in den jeweils ein Klemm-/Wälzkörper 33 (z. B. zylindrischer Klemmkörper) eingelegt ist.

Die (vier) Klauen 29b des zweiten Planetenradträgers (Mitnahmeelements) 29 sind so dimensioniert und winkelbeabstandet, dass sie sich bei einem aktiven Verdrehen (motorisches Antreiben) des Planetenträgers (Mitnahmeelements) 29 gegen die Klauen 34b des Abtriebselements 34 anlegen und gleichzeitig die Wälzkörper 33 im Wesentlichen in der Mitte der planen Flächen 34d des Abtriebselements 34 verklemmungsfrei mitführen. Dieser Zustand ist insbesondere in der Fig. 4, rechte Abbildung dargestellt.

Im Fall eines aktiven Verdrehens (manuelles Antreiben von Seiten des Spannfutters) des Abtriebselements 34 bewegen sich die planen Flächen 34d auf die Wälzkörper 33 zu, bis die Wälzkörper 33 zwischen den planen Flächen 34d und der Hülse 26 verklemmt werden. Dieser Zustand ist insbesondere in der Fig. 4, linke Abbildung dargestellt. Das bedeutet, dass der Planetenradträger (Mitnahmeelement) 29, die Wälzkörper 33 und das Abtriebselement 34 eine Wellenarretierung darstellen, die bei manueller Drehung der Ausgangswelle 30 entgegen dem (ruhenden) Motor die Ausgangswelle 30 mit der Kartusche 16 drehfest verspannt.

Schließlich hat die Ausgangswelle 30 an ihrem distalen Endabschnitt ein Kupplungsstück 35, das aus der Kartusche 16 axial herausragt und mit einem Gegenkupplungsstück, insbesondere der Spannfuttereinheit in Eingriff bringbar ist.

Die Funktion der Getriebeeinheit vorliegend mit integrierter Wellenarretierung lässt sich wie folgt umschreiben.

Die Getriebeeinheit ist für den Betrieb an das Gehäuse der nicht weiter dargestellten Bohr- und/oder Fräsmaschine angeflanscht, wobei die Eingangswelle 18 der Getriebeeinheit mit der (nicht gezeigten) Motorwelle der Maschine in Welleneingriff kommt. Die Ausgangswelle 30 der Getriebeeinheit ist ferner mit der Antriebswelle 1 der Spannfuttereinheit gemäß vorstehender Beschreibung drehfest verbunden.

Wird in diesem Montagezustand eine Drehung vom Antriebsmotor der Maschine auf die Eingangswelle 18 der Getriebeeinheit aufgebracht, wird diese Drehung durch das (Doppel-) Planetengetriebe auf eine gewünschte Drehzahl über-/untersetzt und vom zweiten Planetenradträger (Mitnahmeelement) 29 (über dessen Klauen 29b) auf das Abtriebselement 34 übertragen, wobei die Wälzkörper 33 (mit Spiel) verklemmungsfrei mitgeführt werden. Da das Abtriebselement 34 drehfest auf der Ausgangswelle 30 der Getriebeeinheit sitzt oder mit dieser einstückig ausgebildet ist, dreht die Ausgangswelle 30 entsprechend der über-/untersetzten Drehzahl und treibt so die Spannfuttereinheit an.

Wie vorstehend bei der Beschreibung der Spannfuttereinheit mit Bezug auf einen Werkzeugwechsel erläutert wurde, muss die Antriebswelle 1 der Spannfuttereinheit festgehalten werden, während das Spannfuttergehäuse relativ hierzu (manuell) gedreht wird. D.h., zunächst wird bei einem manuellen Verdrehen des Spannfuttergehäuses für einen Werkzeugwechsel ein Drehmoment (entgegen dem ruhenden Motor) auf die Antriebswelle 1 der Spannfuttereinheit aufgebracht, das über die Ausgangswelle 30 der Getriebeeinheit auf das Abtriebselement 34 übertragen wird. Dieses dreht sich daher geringfügig im Wesentlichen ohne die Mitführung der Wälzkörper 33, d.h., das Abtriebselement 34 dreht sich im Wesentlichen relativ zu den Wälzkörpern 33. Infolge dieser Relativdrehung beabstanden sich die Klauen 29b des zweiten Planetenradträgers (Mitnahmeelements) 29 von den Wälzkörpern 33, wobei auch die beiden planen Flächen (Klemmflächen) 34d am Abtriebselement 34 sich relativ zu den (nunmehr nicht mitgeführten) Wälzkörpern 33 weiterdrehen. Dabei werden die Wälzkörper 33 zwischen den planen Flächen 34d und der Innenwand der Hülse 26 eingeklemmt/verspannt und das Drehmoment unmittelbar über die Wälzkörper 33 auf das Getriebegehäuse (Kartusche 16) übertragen. Da das Getriebegehäuse drehfest mit dem (nicht weiter gezeigten) Maschinengehäuse verflanscht ist, kann ein Benutzer am Maschinengehäusegriff ein Gegendrehmoment aufbringen, ohne umgreifen zu müssen.

D.h. die vorstehend beschriebene Wellenarretierung ist außer Funktion (frei), dann, wenn der zweite Planetenradträger (Mitnahmeelement) 29 vom Motor sowohl für einen Rechts- als auch Linkslauf angetrieben wird und ist in Funktion (arretiert), dann, wenn das Abtriebselement 34 von Seiten der Spannfuttereinheit (manuell) angetrieben wird. Dies erbringt im Betrieb eine Mehrzahl von Vorteilen:

Die Bedienung der beschriebenen schlüssellosen Spannfutteranordnung vorzugsweise bestehend aus der erfindungsgemäßen Spannfuttereinheit und der Getriebeeinheit erfolgt einfach und schnell. Sie ermöglicht ein präzises Arbeiten, da der Anwender den Griff der Schraub-, Bohr-/Fräsmaschine nicht verändern muss, wenn er ein Werkzeug wechselt. Daher ist ein Werkzeugwechsel durch eine einzige Person möglich. Es ist ein festes Einspannen gewährleistet, da die Maschine und die Verschlusshülse optimal ergriffen werden können. Es ergibt sich ferner eine größere Sicherheit dahingehend, dass sich das Werkzeug während der Anwendung auch bei Betrieb in Nebendrehrichtung nicht löst. In Kombination mit dem genannten einhülsigen Dreibackenfutter entsteht ein zuverlässiges System mit geringem Patientenrisiko. Abschließend sei noch explizit darauf hingewiesen, dass durch die integrierte Ausbildung von Getriebeeinheit mit Wellenarretierung die chirurgische Schraub-, Bohr- bzw. Fräsmaschine auch in der Funktion als "herkömmlicher", vorzugsweise in beide Richtungen arbeitender Schraubendreher benutzt werden kann.

Zusammenfassend wird eine schlüssellose Spannfutteranordnung einer medizinischen Schraub-, Bohr- und/oder Fräsmaschine offenbart umfassend eine Spannfuttereinheit mit einem Spannfuttergehäuse, in dem Spannbacken mittels einer Druckspindel axialverschiebbar sind, die mit einer im Spannfuttergehäuse drehbar gelagerten Antriebswelle in Gewindeeingriff steht. Erfindungsgemäß ist ein wahlweise aktivier- und/oder deaktivierbaren Freilauf zwischen der Antriebswelle und dem Spannfuttergehäuse vorgesehen. Zusätzlich oder unabhängig hiervon hat die schlüssellose Spannfutteranordnung eine Getriebeeinrichtung vorzugsweise Planetengetriebeeinrichtung mit integrierter Wellenarretierung, die als externe Einheit zwischen der Schraub-, Bohr- und/oder Fräsmaschine (Schraubendrehermaschine) und der Spannfuttereinheit einsetzbar oder eingesetzt ist.

## Patentansprüche

1. Schlüssellose Spannfutteranordnung einer medizinischen Schraub-, Bohr- und/oder Fräsmaschine mit einem Spannfuttergehäuse (2, 8), in dem Spannbacken (6) mittels einer Druckspindel (4) axialverschiebbar sind, die mit einer im Spannfuttergehäuse (2, 8) drehbar gelagerten Antriebswelle (1) in Gewindeeingriff steht, **dadurch gekennzeichnet, dass** die Spannfutteranordnung weiterhin eine Getriebeeinrichtung mit integrierter Wellenarretierung umfasst, die als externe, separate Einheit ausgebildet und dafür angepasst ist, wahlweise zwischen eine Ausgangswelle der Schraub-, Bohr- und/oder Fräsmaschine und die Antriebswelle (1) im Spannfuttergehäuse (2, 8) eingesetzt zu werden, indem die Einheit an ein Gehäuse der Schraub-, Bohr- und/oder Fräsmaschine anflanschbar ist wobei eine Eingangswelle (18) der Einheit mit der Ausgangswelle der Schraub-, Bohr- und/oder Fräsmaschine in Welleneingriff kommt, wobei die in einem Getriebegehäuse (16, 17) gelagerte Eingangswelle (18) über einen Zahnrad-Getriebezug ein erstes Klauenkupplungsstück (29) antreibt, das mit einem zweiten Klauenkupplungsstück (34) in Kupplungseingriff steht, welches auf einer im Getriebegehäuse (16, 17) gelagerten Ausgangswelle (30) der Getriebeeinrichtung drehfest sitzt oder einstückig mit dieser ausgebildet ist, und wobei der Zahnrad-Getriebezug ein Planetengetriebe ist, mit einem Hohlrad (26), das büchsenförmig in das Getriebegehäuse (16, 17) drehfest eingesetzt ist und gleichzeitig eine Getriebegehäuseseitige Innenwand für Wälzkörper (33) der Wellenarretierung bildet.

2. Schlüssellose Spannfutteranordnung nach Anspruch 1, **dadurch gekennzeichnet, dass** das zweite Klauenkupplungsstück (34) in Umfangsabschnitten zwischen seinen axial vorragenden Klauen (34b) vorzugsweise plane Klemmflächen (34d) ausbildet, die zwischen sich und der Getriebegehäuseseitigen Innenwand jeweils einen im Querschnitt weiter vorzugsweise kreissegmentförmigen Aufnahmeraum definieren, in die jeweils einer der Wälzkörper (33) eingesetzt ist, die sich bei einer aktiven Verdrehung des zweiten Klauenkupplungsstücks (34) relativ zum ersten Klauenkupplungsstück (29) zwischen den jeweiligen Klemmflächen (34d) und der Getriebegehäuseseitigen Innenwand verspannen.

3. Schlüssellose Spannfutteranordnung nach Anspruch 2, **dadurch gekennzeichnet, dass** das erste Klauenkupplungsstück (29) axial vorragende Klauen (29b) hat, die so dimensioniert sind, dass sie bei einer aktiven Verdrehung des ersten Klauenkupplungsstücks (29) relativ zum Getriebegehäuse (16, 17) für ein Antreiben des zweiten Klauenkupplungsstücks (34) die Wälzkörper (33) mitnehmen, um deren Verspannen zwischen den jeweiligen Klemmflächen (34d) und der Getriebegehäuseseitigen Innenwand zu vermeiden.

4. Schlüssellose Spannfutteranordnung nach einem der Ansprüche 1-3, **dadurch gekennzeichnet, dass** das Planetengetriebe ein erstes Sonnenrad (22) hat, das an der Eingangswelle (18) angeordnet ist und mit ersten Planetenrädern (23) kämmt, die am Hohlrad (26) abwälzen und an einem drehbar gehaltenen ersten Planetenradträger (24) gelagert sind, an dem ein zweites Sonnenrad (27) vorgesehen ist, das mit zweiten Planetenrädern (28) in Kämmeingriff steht, die ebenfalls am Hohlrad (26) abwälzen und am ersten Klauenkupplungsstück (29) als weiteren Planetenradträger gelagert sind.

## Claims

1. Keyless clamping chuck arrangement of a medical screwing, drilling and/or milling machine having a clamping chuck housing (2, 8) in which clamping jaws (6) can be axially displaced by means of a pressure spindle (4) which is in threaded engagement with a drive shaft (1) which is rotatably supported in the drill chuck housing (2, 8),
**characterised in that** the clamping chuck arrangement further comprises a gear device having an integrated shaft securing unit which is constructed as an external, separate unit and which is adapted to be inserted selectively between an output shaft of the screwing, drilling and/or milling machine and the drive shaft (1) in the clamping chuck housing (2, 8) by the unit being able to be flanged to a housing of the screwing, drilling and/or milling machine, wherein an input shaft (18) of the unit is in shaft engagement with the output shaft of the screwing, drilling and/or milling machine, wherein the input shaft (18) which is supported in a gear housing (16, 17) drives via a toothed wheel gear train a first claw coupling piece (29) which is in coupling engagement with a second claw coupling piece (34) which is positioned in a rotationally secure manner on an output shaft (30) of the gear device, which shaft is supported in the gear housing (16, 17), or is integrally constructed therewith, and wherein the toothed wheel gear train is a planet gear having a ring gear (26) which is inserted in the manner of a bush into the gear housing (16, 17) in a rotationally secure manner and at the same time forms a gear-housing-side inner wall for roller members (33) of the shaft securing unit.

2. Keyless clamping chuck arrangement according to claim 1, **characterised in that** the second claw coupling piece (34) preferably forms in peripheral portions between the axially protruding claws (34b) thereof planar clamping faces (34d) which between them and the gear-housing-side inner wall define a receiving space which is further preferably circle-segment-like in cross-section and in which there is inserted one of the roller members (33) which with an active rotation of the second claw coupling piece (34) become clamped relative to the first claw coupling piece (29) between the respective clamping faces (34d) and the gear-housing-side inner wall.

3. Keyless clamping chuck arrangement according to claim 2, **characterised in that** the first claw coupling piece (29) has axially protruding claws (29b) which are sized in such a manner that, with an active rotation of the first claw coupling piece (29) relative to the gear housing (16, 17) for driving the second claw coupling piece (34), they carry the roller members (33) in order to prevent the clamping thereof between the respective clamping faces (34d) and the gear-housing-side inner wall.

4. Keyless clamping chuck arrangement according to any one of claims 1 to 3, **characterised in that** the planet gear has a first sun wheel (22) which is arranged on the input shaft (18) and which meshes with first planet wheels (23) which roll on the ring gear (26) and which are supported on a rotatably retained first planet wheel carrier (24), on which there is provided a second sun wheel (27) which is in meshing engagement with second planet wheels (28) which also roll on the ring gear (26) and which are supported on the first claw coupling piece (29) as an additional planet wheel carrier.

## Revendications

1. Dispositif de mandrin sans clé pour une visseuse, perceuse et/ou fraiseuse médicale, avec un boîtier de mandrin (2, 8) dans lequel des mâchoires de serrage (6) sont déplaçables axialement au moyen d'une vis de pression (4) en prise par filetage avec un arbre d'entraînement (1) monté de manière rotative dans le boîtier de mandrin (2, 8),
**caractérisé en ce que** ledit dispositif de mandrin comprend en outre un mécanisme d'engrenage à arrêt d'arbre intégré, lequel est réalisé comme unité externe séparée et est prévu pour être sélectivement monté entre un arbre de sortie de la visseuse, perceuse et/ou fraiseuse et l'arbre d'entraînement (1) dans le boîtier de mandrin (2, 8), l'unité pouvant être bridée sur un boîtier de la visseuse, perceuse et/ou fraiseuse, **en ce qu'**un arbre d'entrée (18) de l'unité vient en prise avec l'arbre de sortie de la visseuse, perceuse et/ou fraiseuse, **en ce que** l'arbre d'entrée (18) monté dans un boîtier d'engrenage (16, 17) entraîne par l'intermédiaire d'un train d'engrenages à pignons une première pièce d'accouplement à griffes (29) en prise avec une deuxième pièce d'accouplement à griffes (34), rapportée de manière solidaire en rotation sur un arbre de sortie (30) du mécanisme d'engrenage monté dans le boîtier d'engrenage (16, 17), ou réalisée d'un seul tenant avec celui-ci, et **en ce que** le train d'engrenages à pignons est un engrenage planétaire, avec une couronne (26) mise en place de manière solidaire en rotation sous forme de douille dans le boîtier d'engrenage (16, 17) tout en formant une paroi intérieure, côté boîtier d'engrenage, pour des corps de roulement (33) de l'arrêt d'arbre.

2. Dispositif de mandrin sans clé selon la revendication 1, **caractérisé en ce que** la deuxième pièce d'accouplement à griffes (34) forme des surfaces de serrage (34d) préférentiellement planes sur des segments périphériques entre ses griffes (34b) axialement saillantes, lesquelles définissent chacune, entre elles-mêmes et la paroi intérieure côté boîtier d'engrenage, un logement à section transversale préférentiellement en segment de cercle, où est mis en place un des corps de roulement (33) qui se serrent en cas de rotation active de la deuxième pièce d'accouplement à griffes (34) par rapport à la première pièce d'accouplement à griffes (29) entre les surfaces de serrage (34d) et la paroi intérieure côté boîtier d'engrenage.

3. Dispositif de mandrin sans clé selon la revendication 2, **caractérisé en ce que** la première pièce d'accouplement à griffes (29) est pourvue de griffes (29b) axialement saillantes dimensionnées de manière à entraîner les corps de roulement (33) en cas de rotation active de la première pièce d'accouplement à griffes (29) par rapport au boîtier d'engrenage (16, 17) pour un entraînement de la deuxième pièce d'accouplement à griffes (34), afin d'éviter le serrage desdits corps de roulement entre les surfaces de serrage (34d) et la paroi intérieure côté boîtier d'engrenage.

4. Dispositif de mandrin sans clé selon l'une des revendications 1 à 3, **caractérisé en ce que** l'engrenage planétaire est pourvu d'un premier pignon solaire (22) disposé sur l'arbre d'entrée (18) et s'engrenant avec des premiers pignons planétaires (23) roulant contre la couronne (26) et montés sur un premier porte-satellites (24) maintenu rotatif, contre lequel est prévue une deuxième roue solaire (27) s'engrenant avec des deuxièmes pignons planétaires (28) roulant également contre la couronne (26) et montés comme autres porte-satellites sur la première pièce d'accouplement à griffes (29).
